# EUROPEAN PATENT APPLICATION

(11) **EP 2 279 703 A1**
(43) Date of publication of application: **02.02.2011**
(21) Application number: 10251344.7
(22) Date of filing: 28.07.2010
(51) Int. Cl.: A61B 17/34

(54) **Surgical portal device including textured surface**

(30) Priority: 29.07.2009 US 229297 P; 13.04.2010 US 758906
(71) Applicant: Tyco Healthcare Group LP, New Haven, CT 06511 (US)
(72) Inventor: Hotter, Josheph, Middletown, CT 06457 (US)
(74) Representative: Soames, Candida Jane

(57) **Abstract**

A surgical portal device (100) having enhanced retention characteristics includes a body portion (110) dimensioned for insertion within a tissue tract. The body portion includes an outer wall (125) defining a longitudinal axis and having a proximal end (112), a distal end (114), and a lumen (115) configured to allow a surgical instrument to pass therethrough. A thread segment (132) extends at least partially along the outer wall of the body portion. At least one of the outer wall and the thread segment has a textured surface (133). The textured surface may define irregularities. The irregularities are dimensioned to engage or receive tissue segments adjacent the tissue tract to thereby facilitate retention of the body portion relative thereto.

## Description

### CROSS REFERENCE TO RELATED APPLICATION

The present application claims the benefit of and priority to U.S. Provisional Application Serial No. 61/229,297 filed on July 29, 2009, the entire contents of which are incorporated herein by reference

### BACKGROUND

### Technical Field

The present disclosure relates to surgical ports. More particularly, the present disclosure relates to surgical access ports having port fixation components to secure the surgical access port relative to tissue of a patient.

### Background of Related Art

Surgical ports, such as introducers, trocars, and cannulas, permit the introduction of a variety of surgical instruments into a body cavity or opening within a patient. In procedures, such as endoscopic, laparoscopic or arthroscopic surgeries, a passage is created through tissue to access an underlying surgical site in the body. A port or cannula is positioned within the passage. Surgical instruments are introduced within the cannula to perform a surgical procedure. It may be advantageous to provide a portal device that can be removably placed within an incision or body opening of a patient to fix the access device therein.

### SUMMARY

Accordingly, a surgical portal device having enhanced retention characteristics is provided. The portal device includes a body portion dimensioned for insertion within a tissue tract. The body portion includes an outer wall defining a longitudinal axis and having a proximal end, a distal end, and a lumen configured to allow a surgical instrument to pass therethrough. A thread segment extends at least partially along the outer wall of the body portion. At least one of the outer wall and the thread segment has a textured surface. The textured surface may define irregularities. The irregularities are dimensioned to engage or receive tissue segments adjacent the tissue tract to thereby facilitate retention of the body portion relative thereto. In one embodiment, the thread segment includes the textured surface. In another embodiment, the outer wall of the body portion includes the textured surface. In yet another embodiment, each of the outer wall of the body portion and the threaded segment includes the textured surface.

The textured surface may include at least one of projections, recesses, barbs, ribs and slots. The textured surface is made via at least one of molding, etching, cutting, co-molding processes.

### BRIEF DESCRIPTION OF THE DRAWINGS

Embodiments of the presently disclosed surgical portal device are described herein with reference to the accompanying drawings, wherein:

FIG. 1 is a perspective view of a surgical portal device according to an embodiment of the present disclosure;

FIG. 2 is a perspective view of a surgical portal device in accordance with another embodiment of the present disclosure;

FIG. 3 is a perspective view of a surgical portal device in accordance with another embodiment of the present disclosure;

FIG. 4 is a perspective view of a surgical portal device in accordance with another embodiment of the present disclosure;

FIG. 5 is a perspective view of a surgical portal device in accordance with another embodiment of the present disclosure; and

FIG. 6 is a detail view of a portion of the surgical portal device of the FIG. 5.

Other features of the present disclosure will become apparent from the following detailed description, taken in conjunction with the accompanying drawings, which illustrate, by way of example, various principles of the present disclosure.

### DETAILED DESCRIPTION OF THE EMBODIMENTS

Particular embodiments of the present disclosure will be described herein with reference to the accompanying drawings. As shown in the drawings and as described throughout the following description, and as is traditional when referring to relative positioning on an object, the term "proximal" refers to the end of the apparatus that is closer to the user and the term "distal" refers to the end of the apparatus that is farther from the user. In the following description, well-known functions or constructions are not described in detail to avoid obscuring the present disclosure in unnecessary detail.

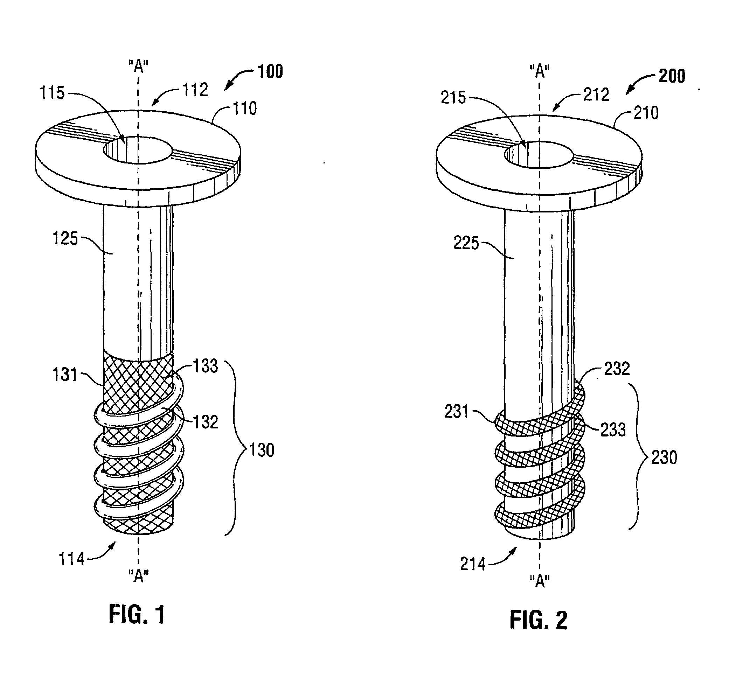

With reference to FIG. 1, a surgical portal device 100 in accordance with embodiments of the present disclosure is shown. The surgical portal device 100 includes a body portion 110 and a threaded portion 130. The body portion 110 includes a proximal end 112, a distal end 114, and a cylindrical bore or lumen 115 extending therethrough and defining a longitudinal axis "A-A." The lumen 115 is dimensioned for reception of at least one surgical instrument (not shown), including, but not limited to, clip appliers, graspers, dissectors, retractors, staplers, laser probes, photographic devices, endoscopes and laparoscopes, tubes, and the like.

The threaded portion 130 includes a plurality of thread segments 132a, 132b, etc. (collectively referred to as "thread segments 132" or "thread segments 132"). As used herein, the term "thread segment" relates to an at least a partial revolution of the threaded portion 130 about the body portion 110. While the term "thread segments" is used to describe portions of the threaded portion 130, "thread segments" is not intended to mean or suggest that the threaded portion 130 is not a single continuous thread. Rather, the threaded portion 130 may include a single continuous thread or a plurality of discontinuous threads. Threaded portion 130 may assist in advancing body portion 110 within a tissue tract via, e.g., by rotating the body portion about longitudinal axis "A-A". Threaded portion 130 also may facilitate retention of the body portion within the tract through engagement with tissue portions defining the tract.

Figures 2-5 illustrate other embodiments of a surgical portal device and are respectively indicated herein as numbers 200, 300, 400 and 500. Surgical portal devices 200, 300, 400 and 500 share many common features as surgical portal device 100 and will not be additionally discussed herein. The differences between each embodiment of surgical portal device 100, 200, 300, 400 and 500 will be discussed herein.

In the embodiments of the present disclosure, various portions of surgical portal device 100, 200, 300, 400, 500 include a respective textured surface 133, 233, 333, 433 and 533. The textured surfaces are configured to enhance or facilitate fixation of the surgical portal device when the surgical portal device is at least partially within tissue. More particularly, the textured surfaces provide an increase in the surface area of the surgical portal device (vis-à-vis a smooth surface) contacting tissue. This increased surface area between surgical portal device and the tissue, e.g., in conjunction with the retention capabilities of the threaded segments results in greater force requirements to remove the surgical portal device from tissue. This feature provides benefits when employing the surgical portal device in a pressurized environment such as, e.g., when used in conjunction with a laparoscopic surgical procedure where the abdominal cavity is insufflated with CO2. Thus, the likelihood of inadvertent movement or retropulsion of the surgical portal device with respect to the tissue is reduced.

In the embodiment illustrated in FIG. 1, the distal portion of an outer surface 125 of body portion 110 includes a textured surface 133. Textured surface 133 is shown extending proximally of threaded portion 130; however textured surface 133 may extend farther proximally or farther distally than illustrated. Additionally, threads 132 of threaded portion 130 are shown having a smooth surface, i.e., without a textured surface.

In the embodiment illustrated in FIG. 2, threads 232 of threaded portion 230 include a textured surface 233, and outer surface 225 of body portion 210 includes a smooth surface.

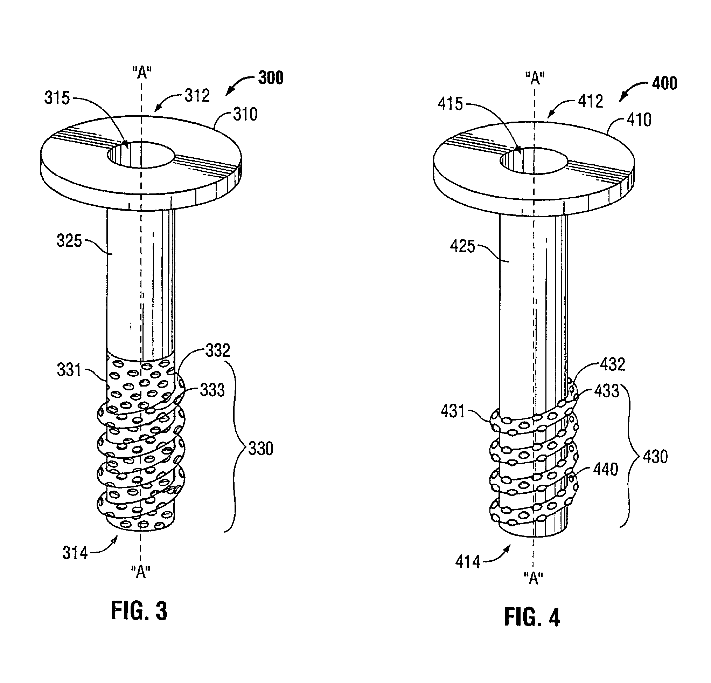

In the embodiment illustrated in FIG. 3, both outer surface 325 of body portion 310 and threads 332 of threaded portion 330 include textured surface 333. In the embodiment illustrated in FIG. 4, threads 432 of threaded portion 430 include a textured surface 433, and outer surface 425 of body portion 410 includes a smooth surface.

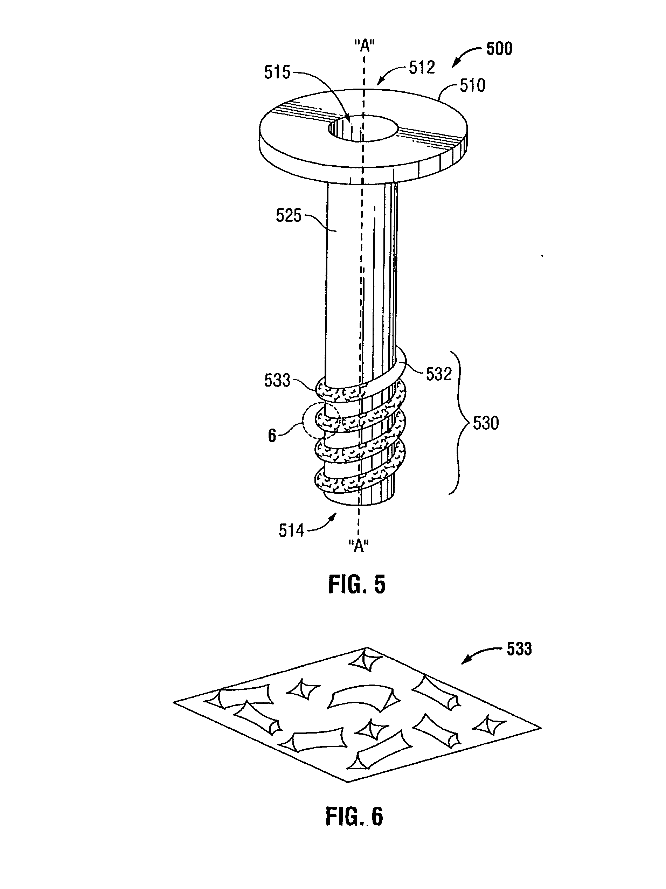

In the embodiment illustrated in FIG. 5, a portion of threads 532 of threaded portion 530 includes a textured surface 533, a portion of threads 532 (i.e., a proximal portion) of threaded portion 530 includes a smooth surface, and outer surface 525 of body portion 510 includes a smooth surface. While a particular portion of threads 532 is textured, it is envisioned that any continuous or discontinuous part of threaded portion 530 includes a textured surface 533.

FIG. 6 is an enlarged view of textured surface 533 in the area indicated in FIG. 5. As shown, textured surfaces 133, 233, 333, 433 and 533 illustrate different types of texture. The types of texture are shown for illustrative purposes only, as body portion and/or threads of each of the illustrated embodiments may include various types of texture. As can be appreciated, the texture can include a repeating pattern, non-repeating pattern, or combinations thereof; the texture can be continuous, discontinuous, or combinations thereof; the texture can include regular shapes, irregular shapes, or combinations thereof; the texture can protrude for a predetermined distance or be recessed a predetermined distance into a portion(s) of the surgical portal device, or combinations of protrusions and recesses thereof.

The texture of textured surfaces 133, 233, 333, 433 and 533 may be applied to the surgical portal device by using a variety of processes. Some of the processes envisioned by the present disclosure include molding, etching, cutting, co-molding, knurling and combinations thereof. For example, textured surfaces on threads may be made by etching the threads in a mold. It is further envisioned that the texture may be applied to the surgical portal device via an additional material being sprayed, coated, or otherwise adhered to the surgical portal device.

It will be understood that various modifications may be made to the embodiments of the presently disclosed surgical portal device. Therefore, the above description should not be construed as limiting, but merely as exemplifications of embodiments. Those skilled in the art will envision other modifications within the scope and spirit of the present disclosure.

## Claims

1. A surgical portal device, comprising:
a body portion dimensioned for insertion within a tissue tract, the body portion including an outer wall defining a longitudinal axis and having a proximal end, a distal end, and a lumen configured to allow a surgical instrument to pass therethrough; and
a thread segment extending at least partially along the outer wall of the body portion, at least one of the outer wall and the thread segment having a textured surface, the textured surface defining irregularities, the irregularities dimensioned to engage or receive tissue segments adjacent the tissue tract to thereby facilitate retention of the body portion relative thereto.

2. The surgical portal device of Claim 1, wherein the thread segment includes the textured surface.

3. The surgical portal device of Claim 1 or Claim 2, wherein the outer wall of the body portion includes the textured surface.

4. The surgical portal device of Claim 1, wherein each of the outer wall of the body portion and the threaded segment includes the textured surface.

5. The surgical portal device of any preceding claim, wherein the textured surface includes at least one of projections, recesses, barbs, ribs and slots.

6. The surgical portal device of any preceding claim, wherein the textured surface is made via at least one of molding, etching, cutting, co-molding.

7. The surgical portal device of any preceding claim, wherein the textured surface of the threaded portion is made by etching the threads in a mold.
